(19) Europäisches Patentamt
European Patent Office
Office européen des brevets

(11) Publication number : **0 308 412 B1**

# (12) EUROPEAN PATENT SPECIFICATION

(45) Date of publication of patent specification : **18.03.92 Bulletin 92/12**

(51) Int. Cl.$^5$ : **C07J 51/00,** C07J 5/00, C07J 13/00, C07J 71/00

(21) Application number : **87903639.0**

(22) Date of filing : **21.05.87**

(86) International application number : **PCT/US87/01153**

(87) International publication number : **WO 87/07612 17.12.87 Gazette 87/28**

## (54) 16-g(a)-METHYLATION PROCESS.

The file contains technical information submitted after the application was filed and not included in this specification

(30) Priority : **04.06.86 US 870364**

(43) Date of publication of application : **29.03.89 Bulletin 89/13**

(45) Publication of the grant of the patent : **18.03.92 Bulletin 92/12**

(84) Designated Contracting States : **AT BE CH DE FR GB IT LI LU NL SE**

(56) References cited :
**EP-A- 0 165 037**
**FR-A- 2 318 647**
**GB-A- 2 001 990**

(73) Proprietor : **THE UPJOHN COMPANY**
**301 Henrietta Street**
**Kalamazoo, Michigan 49001 (US)**

(72) Inventor : **VAN RHEENEN, Verlan, H.**
**2112 Vanderbilt Road**
**Portage, MI 49081 (US)**
Inventor : **HUBER, Joel, E.**
**7267 South 10th Street**
**Kalamazoo, MI 49009 (US)**

(74) Representative : **Perry, Robert Edward et al**
**GILL JENNINGS & EVERY 53-64 Chancery Lane**
**London WC2A 1HN (GB)**

EP 0 308 412 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

Corticoids with a 16α-methyl group are known to be useful anti-inflammatory agents. These include dexamethasone (9α-fluoro-11β,17α-21-trihydroxy-16α-methylpregna-1,4-diene-3,20-dione), flumethasone 6α,9α-difluoro-11β,17α,21-trihydroxy-16α-methylpregna-1,4-diene-3,20-dione) and paramethasone (6α-fluoro-11β,17α,21-trihydroxy-16α-methylpregna-1,4-diene-3,20-dione).

The 16-unsaturated corticoid (I) starting materials are known, see, US Patents 2,864,834, 3,210,341, 3,839,369, 4,031,080 and 4,277,409.

The transformation of 16-unsaturated pregnanes to 16α-methyl pregnanes by use of a Grignard reagent is known. The conjugate addition of a 16α-methyl group to a 16-unsaturated-20-keto steroid by means of a methyl Grignard reagent in the presence of a copper salt catalyst is well known, see Organic Reactions in Steroid Chemistry, Vol. II, J. Fried and J. A. Edwards, Van Nostrand Reinhold Co., NY, 1972, p 75. The product of such a reaction is the 16α-methyl-$\Delta^{17(20)}$-20-enolate, which according to Fried may be trapped as the 20-acetate, see p 76. While the addition can go 1,2 or 1,4 with a ratio of about 1:1, the addition of cuprous chloride gave exclusively 1,4-addition in yields of greater than 90%.

US Patent 3,231,568 (JULIAN) discloses the transformation of a 16-unsaturated progesterone to the corresponding 17α-hydroxy-16α-methylprogesterone. However, JULIAN's pregnane did not have a second α/β-unsaturated ketone in the form of a $\Delta^4$-3-ketone or $\Delta^{1,4}$-3-ketone as the steroid A-ring was 3,5-cyclo. In addition, JULIAN disclosed a progesterone having no $C_{21}$ functionality while the process of the present invention uses a 21-acylate which is sensitive to the Grignard reaction and is hydrolyzable. The C-ring of JULIAN contained no substitution and therefore was not sensitive to the oxidation reaction conditions. A further distinction is that JULIAN traps with acetate and the enol acetate of JULIAN is not reactive to the mild peracid conditions of the present process and hence would not form the desired 17,20-epoxide (III).

US Patent 4,031,080 (PALLADINO) uses copper for the conjugate addition reaction but produces an enol acetate which as JULIAN would teach would be unreactive to the mild and selective peracid reaction conditions of this invention producing the desired 17,20-epoxide (III). PALLADINO produces 17α-bromo-16α-methyl corticoid not a 17α-hydroxy-16α-methyl corticoid.

US Patent 4,277,409 (WARNANT) transforms a 16-unsaturated-21-acetate to a 16α-methyl-21-acetate, without introducing the necessary 17α-hydroxyl group while the process of the present invention introduces the desired 17α-hydroxyl group.

Great Britain Patent 2,001,990 discloses a process to transform a 16-unsaturated corticoid (I) to the corresponding 16α-methyl corticoid (V) by methylating the 16-unsaturated corticoid with copper catalyzed methyl grignard to produce the 16α-methyl-$\Delta^{17(20)}$-20-(magnesium bromide)enolate, followed by conversion by oxygenation to a 17α-hydro-peroxide which is reduced to the corresponding 16α-methyl-17α,21-dihydroxy-20-one 21-acetate. The process of the present invention does not produce a 17α-hydroperoxide, but rather utilizes a 17α,20-epoxide (III) intermediate.

US Patent 3,072,686 (WETTSTEIN) discloses the transformation of a 16-unsaturated progesterone to a 17α-hydroxy-16α-methylprogesterone by reacting the 16-unsaturated progesterone with a Grignard reagent and cuprous chloride. WETTSTEIN produces a $\Delta^{17(20)}$-20-enol acetate which is not reactive to the mild and selective peracid conditions of the present process and hence would not form the desired 17,20-epoxide (III).

Both J. Am. Chem. Soc. 80, 3160 (1958) and J. Am. Chem. Soc. 80, 4428 (1958) report the transformation of a 16-unsaturated progesterone to a 16α-methylprogesterone by use of a Grignard reagent followed by peracid oxidation to introduce the 17α-hydroxy group producing a 17α- hydroxy-16α-methylprogesterone. The 17α-hydroxy-16α-methylprogesterone is brominated and acylated to form the 21-acetoxy-17α-hydroxy-16α-methyl steroid.

E. J. Corey in Tetrahedron Letters 26, 6019 (1985), [COREY], discloses the reaction of a methylating agent (lithium dimethylcuperate) with an α,β-enone in the presence of a trapping agent (chlorotrimethylsilane). The significant difference between COREY and the present patent application is that COREY used preformed alkyl-cuperate where as the present invention generates the cuperate methylating agent in situ. Likewise, A. Alexakis in Tetrahedron Letters 27, 1047 (1986), [ALEXAKIS], discloses the reaction of lithium alkyl-cuperate with unsaturated esters in the presence of trimethylsilyl chloride and demonstrates an increased yield of the alkylated product. However, again ALEXAKIS like COREY used preformed cuperate whereas the present invention generates the cuperate methylating agent in situ.

US Patent 3,700,660 discloses a process of converting a 20-acyloxy-17,20-epoxy steroid to a 17α-acyloxy-20-keto steroid by use of a strong acid. US Patent 3,700,660 (HEMPEL) uses 20-acetate where the process of the present invention uses 20-sylyl. The significance of this is that the $\Delta^{7(20)}$-20-enol silane (II) of the

present invention is much more reactive than the corresponding 20-acylate of HEMPEL which permits epoxidation of the $\Delta^{7(20)}$ double bond in steroids having other double bonds such as $\Delta^{4}$-3-keto, $\Delta^{1,4}$-3-keto, $\Delta^{9(11)}$-etc. whereas the 20-acylate of HEMPEL is limited to a non-functionalized steroid.

US Patents 3,513,163 and 4,036,831 disclose $C_{21}$ and $C_{11}$ trimethyl-siloxy ethers respectively. $\Delta^{17(20)}$-20-0-substituted steroids are known where the substituent is an acyl group or a Grignard substituent (-Mg-X), see US Patents 3,072,686, 3,231,568 and 4,031,080. This Mg-X substituted compound while disclosed is not isolatable.

17α,20-Epoxy-16α-methyl-20-0-substituted steroids are disclosed in US Patent 3,876,633 where the 20-0-substituent is an acyl group, see Claim 9.

Selective epoxidation of the $\Delta^{9(11)}$ double bond over the $\Delta^{16}$ double bond in a $\Delta^{9(11),16}$-diene is set forth in US Patent 3,876,633. US Patent 3,876,633 discloses 9β,11β-epoxy-$\Delta^{16}$-steroids where the A-ring is reduced. 9β,11β-epoxy-6α-fluoro-21-hydroxypregna-1,4,16-triene-3,20-dione 21-acetate is disclosed in US Patent 3,210,341, Example 9(b).

US Patent 4,036,831 discloses a process of protecting the 11β-hydroxyl group of a steroid, during subsequent reactions, with trimethylsilyl and the subsequent removal of the trimethylsilyl group by hydrolysis with 40-60% aqueous hydrogen fluoride.

EP-A-0165037 discloses the preparation of a $\Delta^{17(20)}$-steroid (II A-C), which comprises (1) contacting a 16-unsaturated corticoid (I A-C) with a methylating agent in the presence of a copper catalyst and (2) contacting the product of step (1) with a silylating agent.

## SUMMARY OF THE INVENTION

A novel process for the preparation of a $\Delta^{17(20)}$-steroid (II A-C) comprises contacting the 16-unsaturated corticoid (I A-C) with a methylating agent in the presence of a copper catalyst and a silylating agent.

As shown in Chart A, the product can be converted to a desired 16α-methyl corticoid (V). 16α-methyl corticoids (V) are adrenocorticoid agents with glucocorticoid activity and are useful primarily for their anti-inflammatory effect, as is well known to those skilled in the art. They include dexamethasone, flumethasone and paramethasone. Dexamethasone is one of the best known 16α-methyl corticoids; see US-A-3375261 and US Reissue Patent 28,369, as well as the Physicians Desk Reference 1983, 37th Edition, p. 1270-1283.

## DETAILED DESCRIPTION OF THE INVENTION

The 16-unsaturated corticoid (I A-C) starting materials are well known to those skilled in the art or can be readily prepared from known steroids by methods well known to those skilled in the art, see, for example, US Patents 2,773,080, 2,864,834, 3,210,341, 3,441,559, 3,461,144, 3,493,563, 3,839,369, 4,031,080, and 4,277,409.

The $C_3$ functionality of the $\Delta^{4}$-3-keto (A), $\Delta^{1,4}$-3-keto (B) and 3β-hydroxy-$\Delta^{5}$- (C) steroid does not have to be protected for the processes of the present invention. The 3β-hydroxy-$\Delta^{5}$- (C) steroid can have its $C_3$-hydroxyl group (Ca) protected as the silyl ether (Cb), ether (Cc) or ester (Cd), see Chart C. The free hydroxyl group (Ca) can be protected as the ether (Cc) or ester (Cd) as is well known to those skilled in the art. See Protective Groups in Organic Synthesis, Theodora W. Greene, Wiley & Sons, New York 1981. The ethers (Cc) are prepared by methods well known to those skilled in the art, see Steroid Reactions, Edited by Carl Djerassi, Holden-Day, San Francisco 1967, p 76-82. If the free 3β-hydroxyl group (Ca) is not protected as the ether (Cc) or ester (Cd) during the silation reaction, the free hydroxy group will be silated to form the silyl ether (Cb). During the silylation reaction, if the 3β-hydroxyl group is free and will be silylated, one additional equivalent of Grignard and silylating agent will be consumed. The $C_3$ protected forms of the 3β-hydroxy steroids (C) are considered equivalent to the non-protected or free form (C) respectively since the $C_3$ protecting groups are readily removable to convert the $C_3$ protected forms (Cb, Cc and Cd) to (A and C). The protecting group remains on until the hydrolysis (acid or base) of the 17α,20-epoxide. If acid hydrolysis is utilized and the $C_3$ protecting group is acid-labile (Cb and Cc) it will be removed. Likewise if base hydrolysis is utilized and the $C_3$ protecting group is base-sensitive (Cd) it will be removed. If the $C_3$ protecting group is not sensitive to the hydrolysis agent, the $C_3$ protected steroid will have to be treated with the appropriate agent to remove the $C_3$ protecting group.

It is preferred that the 16-unsaturated corticoid (I A-C) be a $\Delta^{4}$-3-keto (A) or a $\Delta^{1,4}$-3-keto (B) corticoid, more preferably a $\Delta^{1,4}$-3-keto (B) corticoid. It is preferred that the 16-unsaturated corticoid have the C-ring $\Delta^{9(11)}$ or 9β,11β-epoxy. It is more preferred that the C-ring be 9β,11β-epoxy. It is preferred that $R_6$ be a hydrogen or fluorine atom, more preferred that $R_6$ be a hydrogen atom.

While it is preferred that the C-ring is $\Delta^{9(11)}$ or 9β,11β-epoxy, the C-ring can be converted to the desired 9α-fluoro-11β-hydroxy functionality prior to the Grignard addition of the methyl group to the $\Delta^{16}$ double bond.

If this is done, the 11β-hydroxy group should be protected as is well known to those skilled in the art, see for example, US Patent 4,036,831 where the protecting group is trimethylsilyl. Following the formation of the desired 16α-methyl corticoid, the (trimethylsilyl) protecting group is removed as is well known to those skilled in the art, see for example, US Patent 4,036,831.

The conjugate addition of a methylating agent, such as methyl Grignard, to a 16-unsaturated steroid to give the corresponding 16α-methyl steroid is well known, see Organic Reactions in Steroid Chemistry, Vol. II, J. Fried and J. A. Edwards, p 75 and US Patent 3,072,686.

The methylating agent is selected from the group consisting of $CH_3Cu$, $(CH_3)_2CuM$ or $CH_3MgQ$ and a catalytic amount of a copper (cupric) salt. The preferred methylating agent is methyl Grignard, preferably methyl magnesium chloride. The copper salt can be a cuprous salt such as cuprous chloride, bromide, iodide or cyanide or a cupric salt such as cupric chloride, cupric acetate, cupric propionate or complexes thereof. Examples of copper complexes include, cuprous bromide dimethylsulfide, cuprous chloride tris-n-butylphosphine and cuprous acetylacetonate. The nature of the copper complex is not critical. Hundreds (or thousands) of copper complexes are known which are considered equivalent to those set forth above. Preferred is cupric acetate or propionate, more preferred is cupric propionate. Additional catalysts which are considered equivalent to those disclosed above are well known to those skilled in the art, see, for example, Alfa Catalog, 1983-1984, Morton Thiokol, Inc., Alpha Products, PO Box 299, 152 Andover Street, Danvers, Mass. 01923. Depending on the physical facilities, it may be preferred that the cupric propionate be predissolved. Solvents suitable for the methylation reaction include those selected from the group consisting of THF, t-butylmethyl ether or dimethoxyethane. The reaction is performed in a range of from about -50° to about 20°, preferably at about -20°. When TLC indicates that no starting material is left (indicating the probable formation of an enolate intermediate), the silating agent is added and the resulting product is the $\Delta^{17(20)}$ steroid (II). After the silylating agent is added the reaction temperature is kept in the range of about -25° to about 25°, preferably about 0°. It is preferable to filter the Grignard reaction mixture to prevent residual copper from entering the peracid reaction.

In the present invention the enolate intermediate is trapped with a trapping (silylating) agent which gives the $\Delta^{17(20)}$-20-(substituted silyl) product. Operable silylating agents include $(R_{20})_3$-si-E-, bistrimethylsilylacetamide. It is preferred that the silating agent be of the formula $(R_{20})_3$-Si-E, more preferably trimethylsilyl chloride. Additional silylating agents considered equivalent to those disclosed above are well known to those skilled in the art, see, for example Silicon Compounds, Petrarch Systems, Inc., Bartram Rd. Bristol, PA 19007.

The trapping of the enolate intermediate to produce the $\Delta^{17(20)}$-20-(acetate) is known, see US Patent 4,031,080 and Organic Reactions in Steroid Chemistry, Vol. II, supra, p 76. These enol acylates are too unreactive to react selectively over A,B,C-ring functionality such as $\Delta^{9(11)}$. However, surprisingly and unexpectedly the $\Delta^{17(20)}$-20-(substituted silyl) derivative (II) is sufficiently reactive to react with electrophiles without affecting most of the other functionality in the A,B,C--rings.

The $\Delta^{17(20)}$-steroid (II) can be isolated if desired by means well known to those skilled in the art. However, since the desired product is the 16α-methyl corticoid (V), it is not necessary and preferable not to isolate the $\Delta^{17(20)}$-steroid (II) but rather to continue the reaction.

The $\Delta^{17(20)}$-steroid (II) is reacted with a peracid to produce the 17α,20-epoxide (III). While most peracids are operable, preferred peracids include m-chloroperbenzoic, perbenzoic, peracetic. The peracid-reaction conditions are well known to those skilled in the art. The peracid reaction does not significantly affect the $\Delta^1$, $\Delta^4$, or $\Delta^{9(11)}$ functionalities in the remainder of the molecule. The solvent used during the methylation reaction producing the $\Delta^{17(20)}$steroid (II) is removed and replaced by a non-polar solvent such as toluene, methylene chloride, ethyl acetate or t-butyl methyl ether. The inorganic salts remaining after the methylation reaction are removed by extraction. The peracid oxidation is performed in the temperature range of about -30° to 25°, preferably about -20°. When the reaction is complete, the excess peracid is destroyed by addition of an agent such as powdered sodium thiosulfate or sodium bisulfite. The 17α,20-epoxide (III) can be isolated if desired by means well known to those skilled in the art. However, since the desired product is the 16α-methyl corticoid (V), it is not necessary and preferable not to isolate the 17αa,20-epoxide (III) but rather to continue the reaction in situ.

The 17α,20-epoxide (III) is transformed to the corresponding 16α- methyl corticoid (V) by acid or base hydrolysis. If base is used the 16α-methyl corticoid will be obtained as the 21-hydroxy compound ($R_{21}$ is a hydrogen atom). If acid is used the 16α-methyl corticoid will be the 21-ester ($R_{21}$ is -CO-$R_{21}'$). Suitable base hydrolyzing agents include hydroxide, carbonate, bicarbonate, alkoxide in alcohol at low temperature etc. It is preferred that the hydrolyzing agent be an acid. Suitable acids include mineral acids and other sufficiently strong acids such as p-toluene-sulfonic acid monohydrate, or sulfuric, hydrochloric, citric or acetic acid. The acid hydrolysis is sufficiently fast that the reaction is complete in about 1/2 hour at 20-25°.

If the C-ring of the 16α-methyl corticoid (V) is $\Delta^{9(11)}$ or 9β,11β-epoxy, it is readily convertible to the pharmacologically active 9α-fluoro-11β-hydroxy C-ring by means well known to those skilled in the art.

DEFINITIONS

The following definitions are for the terms as used throughout the description.

All temperatures are in degrees Centrigrade.

TLC refers to thin-layer chromatography.

THF refers to tetrahydrofuran.

$R_3$ , $R_3'$, $R_6$, $R_9$, $R_{11}$, $R_{20}$, $R_{21}$, $R_{21}'$, $R_{121}$, ~, ...., TMS, THP and EEE are as defined in claim 2.

M and Q are as defined in claim 3.

E, Rα and Rβ are as defined in claim 7.

The following Example illustrates the invention.

Example 9β,11β-Epoxy-17β,21-dihydroxy-16α-methylpregna-1, 4-diene-3,20-dione 21-acetate (VB)

A mixture of copper (II) propionate (802 mg) in THF (90 ml) and tetramethylurea (5 ml) is cooled to -25° under nitrogen and treated with about 3 ml of methyl magnesium chloride (2 M) in order to convert the blue copper (II) to the yellow-green copper (I) species. Then methylene chloride (90 ml), trimethylsilyl chloride (6.56 ml) and 9β,11β-epoxy-21-hydroxypregna-1,4,16-triene-3,20-dione 21-acetate (IB, Example 7 of EP-A-0165037, 15.30 g) are added and the temperature is lowered to -33°. A total of 28 ml of methyl magnesium chloride is added to the slurry over a 2.0 hr period. The temperature is maintained in the range of -33° to -38° during this time. When TLC indicates that reaction to the $\Delta^{17(20)}$-corticoid silyl ethyl (II) is complete, the mixture is treated at -10° with ammonium chloride (0.5 M, 200 ml). The layers are separated and the organic layer is washed with water (200 ml). The separate aqueous layers are extracted with methylene chloride (2 x 50 ml). The organic extracts are combined and concentrated to give the corresponding $\Delta^{17(22)}$-corticoid silyl ether.

This material is dissolved in toluene (100 ml). Sodium acetate (1.0 g) followed by peroxyacetic acid (34%, 11.8 ml) are added at 16°. The mixture is stirred at -16 to 10° over 19 hr following which the excess peracid si quenched with sodium bisulfite (1 M). Water (100 ml) is added and the layers are separated. The aqueous layer is extracted with toluene (75 ml). The organic layers are washed sequentially with water (2 x 75 ml) and then combined, filtered thru cotton and concentrated. The concentrate is dissolved in methanol and reconcentrated to remove all the toluene and give the corresponding 17α,20-epoxide.

The 17α,20-epoxide is dissolved in methylene chloride (40 ml) and methanol (70 ml) under nitrogen. The temperature is adjusted to 40° and a solution of potassium bicarbonate (200 mg) and potassium carbonate (200 mg) in water (5 ml) is added. This mixture is stirred at reflux (about 42°). After 75 min, TLC indicates both the $C_{20}$ trimethylsilyl and $C_{21}$ acetate groups have been removed. The reaction is stopped by addition of acetic acid (0.6 ml) and the mixture concentrated at ordinary pressure to about 50 ml. The resulting slurry is cooled to about 0° and the solids are collected, washed with cold methanol and dried under reduced pressure at 60° to give the title compound, mp 260° with dec.

## CHART A

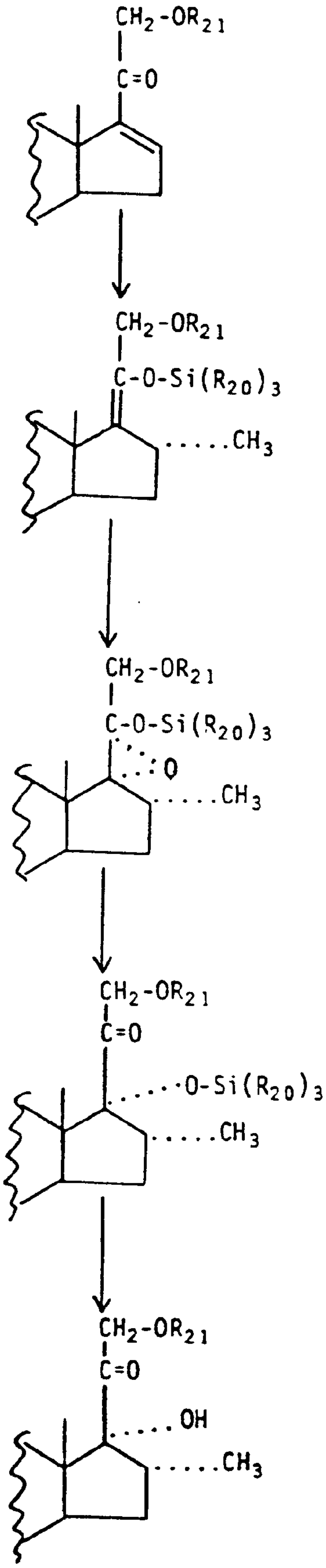

(I)

(II)

(III)

(IV)

(V)

CHART B

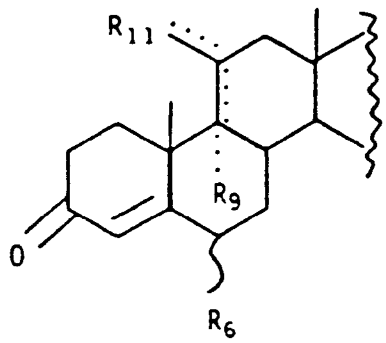

(A)

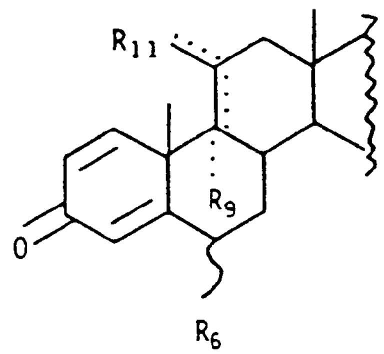

(B)

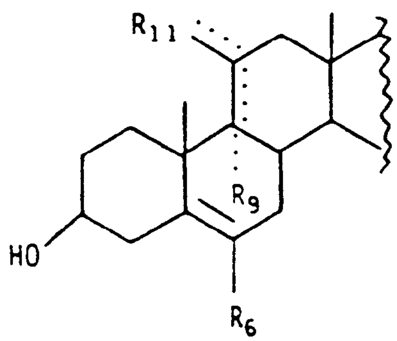

(C)

CHART C

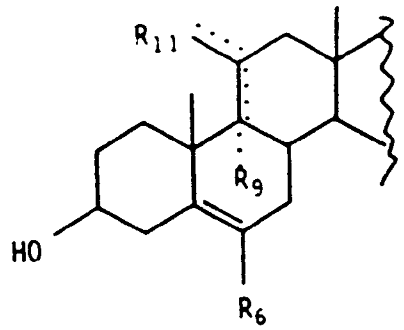

(Ca)

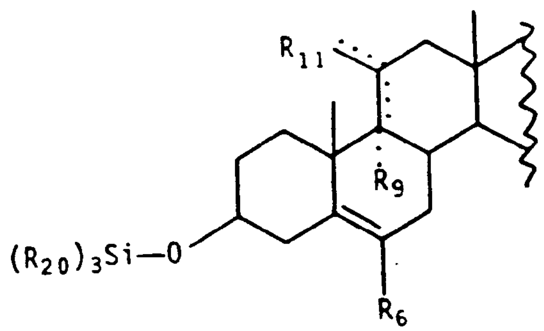

(Cb)

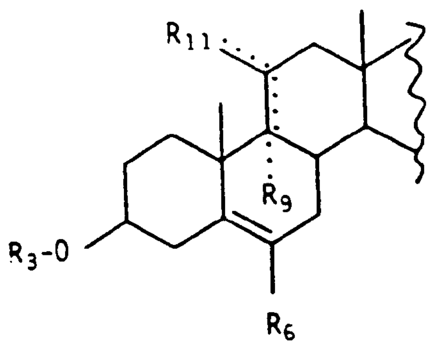

(Cc)

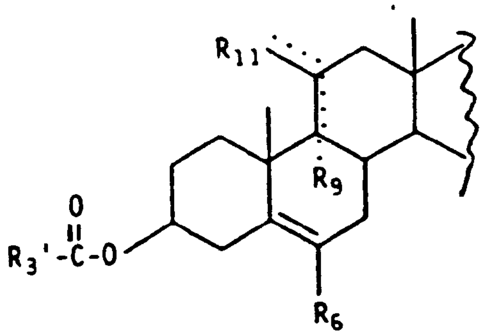

(Cd)

## Claims

1. A process for the preparation of a $\Delta^{17(20)}$-steroid of the formula

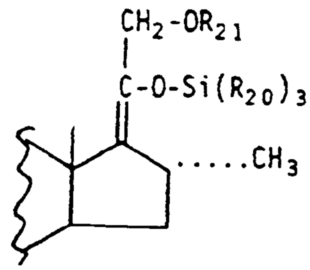

(II)

which comprises contacting a 16-unsaturated corticoid of the formula

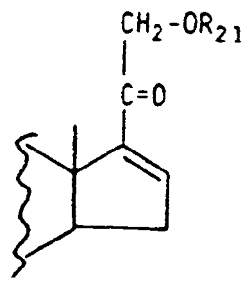

(I)

with a methylating agent in the presence of a copper catalyst and a silylating agent capable of introducing the group $Si(R_{20})_3$, where
$R_{20}$ is alkyl of 1 thru 4 carbon atoms or phenyl (the $R_{20}$'s can be the same or different); and
$R_{21}$ is a hydrogen atom, $-COOR_{21}'$ or $-Si(R_{121})_3$ in which $R_{21}$ and $R_{121}$ are each alkyl of 1 thru 4 carbon atoms or phenyl (the $R_{121}$'s can be the same or different).

2. A process according to claim 1 where the 16-unsaturated corticoid (I) is selected from the group consisting of $\Delta^4$-3-keto steroids

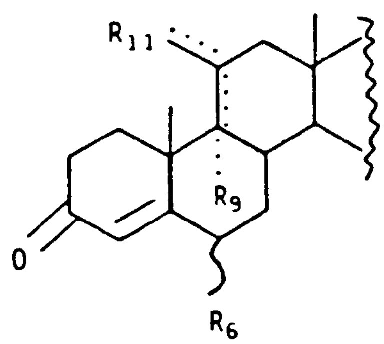

(A)

$\Delta_{1,4}$-3-keto steroids

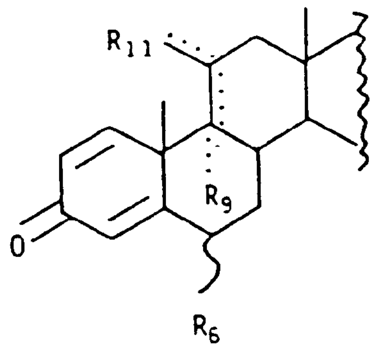

(B)

and 3β-hydroxy-$\Delta^5$-steroids

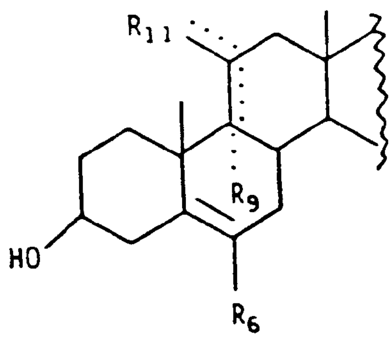

(C)

and the $C_3$ protected forms of the 3β-hydroxy-$\Delta^5$-(C)-steroid

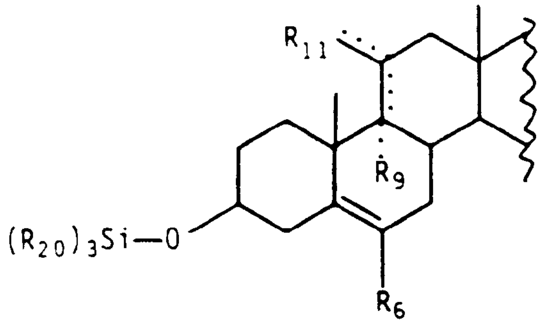

(Cb)

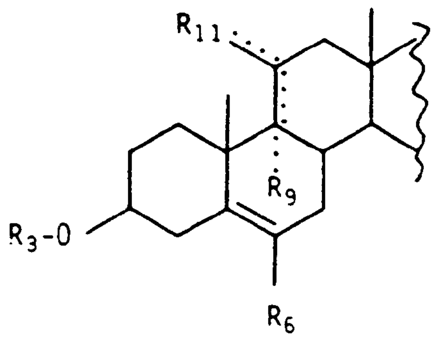

(Cc)

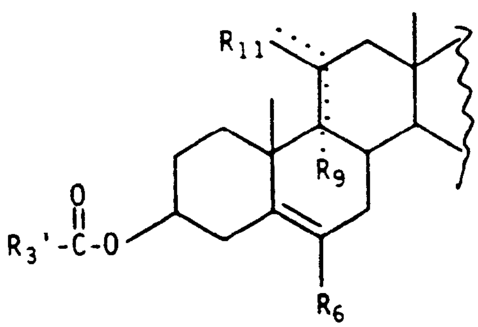

(Cd)

where

$R_3$ is alkyl of 1 thru 3 carbon atoms, a TMS, THP, or EEE group.

$R_3'$ is alkyl of 1 thru 5 carbon atoms or phenyl.

$R_6$ is a hydrogen or fluorine atom or methyl group.

$R_9$ is nothing, a hydrogen, fluorine or oxygen atom which makes the C-ring

a) $\Delta^{9(11)}$ when $R_9$ is nothing and

b) 9β,11β-epoxide when $R_9$ and $R_{11}$ taken together are an oxygen atom.

$R_{11}$ is a hydrogen or oxygen atom, two hydrogen atoms, or α- or β-hydroxyl group or trimethylsilyl ether thereof which makes the C-ring

a) $\Delta^{9(11)}$ when $R_{11}$ is a hydrogen atom,

b) 9β,11β-epoxide when $R_9$ and $R_{11}$taken together are an oxygen atom and .... between $C_{11}$ and $R_{11}$ is a single bond, and

c) a ketone when $R_{11}$ is an oxygen atom and .... between $C_{11}$ and $R_{11}$ is a double bond.

$R_{20}$ is alkyl of 1 thru 4 carbon atoms or phenyl, the $R_{20}$'s can be the same or different.

$R_{21}$ is a hydrogen atom, -CO-$R_{21}$' or -Si($R_{121}$)$_3$.

$R_{21}$' is alkyl of 1 thru 4 carbon atoms or phenyl.

$R_{121}$ is alkyl of 1 thru 4 carbon atoms or phenyl, the $R_{121}$'s can be the same or different.

~ indicates that the attached group can be in either the α or β configuration;

.... is a single or double bond;

TMS refers to trimethylsilyl;

THP refers to tetrahydropyranyl;

EEE refers to (1-ethoxy) ethyl ether [-O-CH($CH_3$)O$CH_2CH_3$].

3. A process according to Claim 1 where the methylating agent is selected from the group consisting of compounds of the formula $CH_3Cu$, $(CH_3)_2CuM$ or $CH_3MgQ$ where M is a lithium or magnesium ion and Q is a chlorine, bromine or iodine atom.

4. A process according to Claim 3 where the methylating agent is $CH_3MgQ$.

5. A process according to Claim 1 where the copper catalyst is a cupric salt selected from the group consisting of cupric acetate, cupric chloride, cupric sulfate, or a cuprous salt selected from the group consisting of cuprous chloride, cuprous bromide, cuprous iodide and cuprous cyanide and complexes thereof.

6. A process according to Claim 1 where the copper catalyst is cupric acetate.

7. A process according to Claim 1 where the silylating agent is selected from the group consisting of $(R_{20})_3$-Si-E, bistrimethylsilyl acetamide, where E is a chlorine, bromine or iodine atom or -NRαRβ where Rα and Rβ may be the same or different and are alkyl of 1 thru 5 carbon atoms or phenyl and where Rα and Rβ can be connected or cyclized in a ring with or without an oxygen or additional nitrogen atom and where $R_{20}$ is defined in Claim 1.

8. A process according to Claim 1 where the silylating agent is trimethylsilyl chloride.

9. A process according to Claim 1 where the 16-unsaturated corticoid (I) is selected from the group consisting of 21-hydroxypregna1,4,9(11),16-tetraene-3,20-dione 21-acetate or 9β,11β-epoxy-21-hydroxypregna-1,4,16-triene-3,20-dione 21-acetate.

10. A process according to claim 1 where the silylating agent is present when the enolate intermediate is formed.

## Patentansprüche

1. Verfahren zur Herstellung eines $\Delta^{17(20)}$-Steroids der Formel

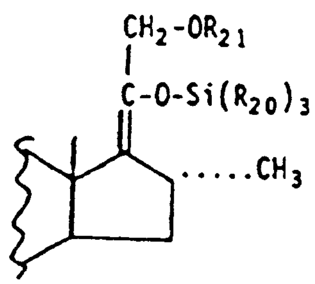

(II)

welches das Kontaktieren eines 16-ungesättigten Corticoids der Formel

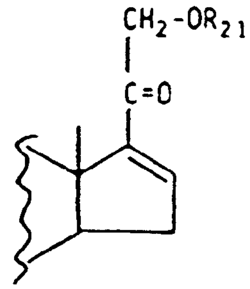

(I)

mit einem Methylierungsmittel in Gegenwart eines Kupferkatalysators und eines Silylierungsmittels, welches fähig ist, die $Si(R_{20})_3$-Gruppe, worin $R_{20}$ Alkyl mit 1 bis 4 Kohlenstoffatomen oder Phenyl bedeutet (wobei die $R_{20}$-Reste gleich oder voneinander verschieden sein können); und

$R_{21}$ ein Wasserstoffatom, $-COOR_{21}'$ oder $-Si(R_{121})_3$ bedeutet, worin $R_{21}'$ und $R_{121}$ jeweils Alkyl mit 1 bis 4 Kohlenstoffatomen oder Phenyl bedeutet (wobei die $R_{121}'$-Reste gleich oder voneinander verschieden sein können), einzuführen, umfaßt.

2. Verfahren nach Anspruch 1, worin das 16-ungesättigte Corticoid (I) aus der Gruppe bestehend aus den $\Delta^4$-3-Ketosteroiden

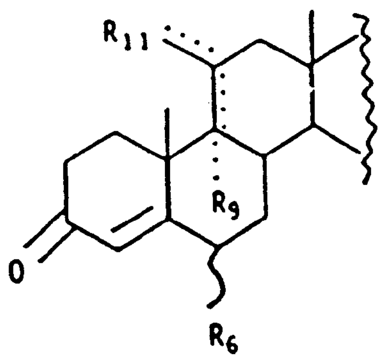

(A)

$\Delta^{1,4}$-3-Ketosteroiden

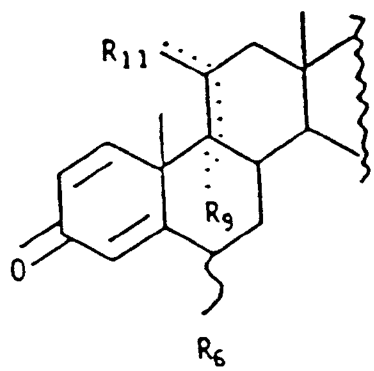

(B)

und 3β-Hydroxy-$\Delta^5$-steroiden

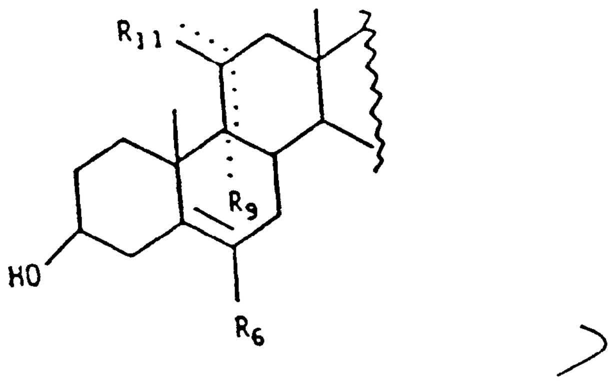

(C)

und den $C_3$ geschützten Formen der 3β-Hydroxy-$\Delta^5$-(C)-steroiden

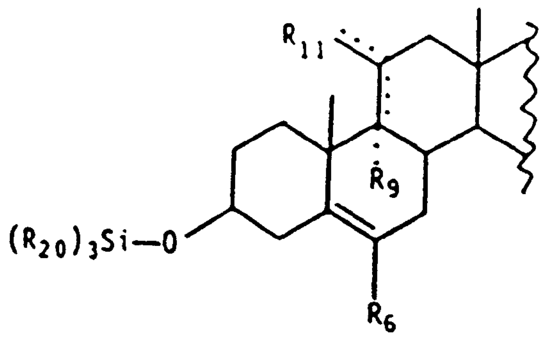

(Cb)

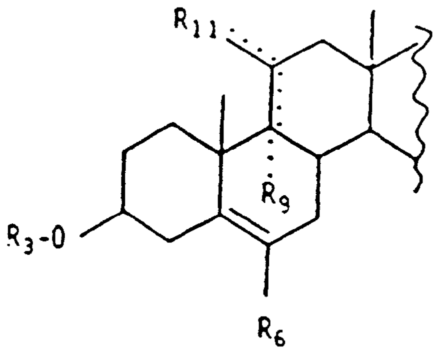

(Cc)

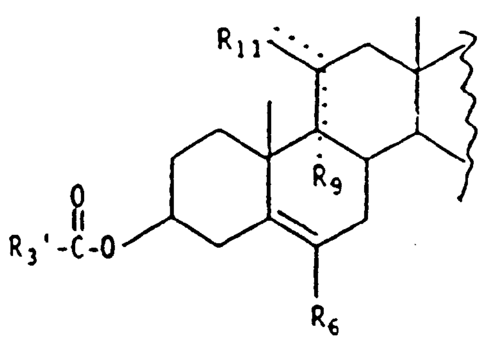

(Cd)

worin

$R_3$ Alkyl von 1 bis 3 Kohlenstoffatomen, eine TMS-, THP-oder EEE-Gruppe bedeutet,

$R_3'$ Alkyl mit 1 bis 5 Kohlenstoffatomen oder Phenyl bedeutet,

$R_6$ ein Wasserstoff- oder Fluoratom oder eine Methylgruppe bedeutet,

$R_9$ nicht vorhanden ist, ein Wasserstoff-, Fluor- oder Sauerstoffatom, welches den C-Ring

a) $\Delta^{9(11)}$ bedeutet, wenn $R_9$ nicht vorhanden ist, und

b) 9β,11β-Epoxid, wenn $R_9$ und $R_{11}$ gemeinsam ein Sauerstoffatom darstellen, bildet, bedeutet,

$R_{11}$ ein Wasserstoff- oder Sauerstoffatom, zwei Wasserstoffatome oder α- oder β-Hydroxylgruppe oder Trimethylsilylether davon, bedeutet, welcher den C-Ring

a) $\Delta^{9(11)}$ wenn $R_{11}$ ein Wasserstoffatom ist,

b) 9β,11β-Epoxid, wenn $R_9$ und $R_{11}$ gemeinsam ein Sauerstoffatom bedeuten und ... zwischer. $C_{11}$ und $R_{11}$ eine Einfachbindung bedeutet, und

c) ein Keton, wenn $R_{11}$ ein Sauerstoffatom bedeutet und ... zwischen $C_{11}$ und $R_{11}$ eine Doppelbindung bedeutet, bildet, $R_{20}$ Alkyl mit 1 bis 4 Kohlenstoffatomen oder Phenyl bedeutet, die

$R_{20}$-Reste können gleich oder voneinander verschieden sein,

$R_{21}$ ein Wasserstoffatom, -CO-$R_{21}'$ oder $Si(R_{121})_3$ bedeutet,

$R_{21}$ Alkyl mit 1 bis 4 Kohlenstoffatomen oder Phenyl bedeutet,

$R_{121}$ Alkyl mit 1 bis 4 Kohlenstoffatomen oder Phenyl bedeutet, die $R_{121}'$-Reste können gleich oder voneinander verschieden sein, - zeigt, daß die gebundene Gruppe entweder die α - oder β-Konfiguration besitzen kann,

... eine Einfach- oder Doppelbindung ist;

TMS Trimethylsilyl bedeutet;

THP Tetrahydropyranyl bedeutet;

EEE (1-Ethoxy)ethylether [$-O-CH(CH_3)OCH_2CH_3$] bedeutet.

3. Verfahren nach Anspruch 1, worin das Methylierungsmittel aus der Gruppe bestehend aus den Formeln $CH_3Cu$, $(CH_3)_2CuM$ oder $CH_3MgQ$, worin M ein Lithium- oder Magnesiumion bedeutet und Q ein Chlor-, Brom- oder Iodatom ist, gewählt ist.

4. Verfahren nach Anspruch 3, worin das Methylierungsmittel $CH_3MgQ$ ist.

5. Verfahren nach Anspruch 1, worin der Kupferkatalysator ein Kupfersalz gewählt aus der Gruppe bestehend aus Kupfer(II)acetat, Kupfer(II)chlorid, Kupter(II)sulfat oder aus der Gruppe bestehend aus Kupfer(I)chlorid, Kupfer(I)bromid, Kupfer(I)iodid und Kupfer(I)cyanid und Komplexen davon, ist.

6. Verfahren nach Anspruch 1, worin der Kupferkatalysator Kupfer(II)acetat ist.

7. Verfahren nach Anspruch 1, worin das Silylierungsagens aus der Gruppe bestehend aus $(R_{20})_3$-Si-E, Bistrimethylsilylacetamid, worin E ein Chlor-, Brom- oder Iodatom bedeutet, oder -NRα Rβ, worin R α und Rβ gleich oder voneinander verschieden sind, und Alkyl von 1 bis 5 Kohlenstoffatomen oder Phenyl bedeuten, und worin R α und Rβ verbunden oder in einen Ring mit oder ohne ein Sauerstoff- oder zusätzliches Stickstoffatom cyclisiert werden können, und worin $R_{20}$ wie in Anspruch 1 definiert ist.

8. Verfahren nach Anspruch 1, worin das Silylierungsmittel Trimethylsilylchlorid ist.

9. Verfahren nach Anspruch 1, worin das 16-ungesättigte Corticoid (I) aus der Gruppe bestehend aus 21-Hydroxypregna-1,4,9(11),16-tetraen-3,20-dion-21-acetat oder 9β,11β-Epoxy--21-hydroxypregna-1,4,16-trien-3,20-dion-21-acetat gewählt ist.

10. Verfahren nach Anspruch 1, worin das Silylierungsmittel vorhanden ist, wenn das Endzwischenprodukt gebildet wird.

## Revendications

1. Procédé de préparation d'un $\Delta^{17(20)}$-stéroïde de formule

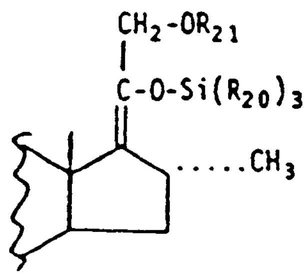

(II)

qui consiste à faire entrer un corticoïde non saturé en position 16, de formule

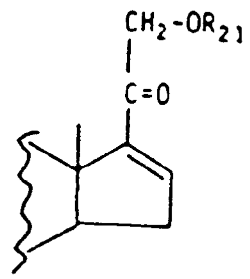

( I )

en contact avec un agent de méthylation en présence d'un catalyseur au cuivre et d'un agent de silylation capable d'introduire le groupe $Si(R_{20})_3$, où

$R_{20}$ est un radical alkyle ayant 1 à 4 atomes de carbone ou phényle (les radicaux $R_{20}$ peuvent être identiques ou différents) ; et

$R_{21}$ est un atome d'hydrogène, un groupe $-COOR_{21}'$ ou $-Si(R_{121})_3$ où $R_{21}'$ et $R_{121}$ représentent chacun un groupe alkyle ayant 1 à 4 atomes de carbone ou phényle (les groupes $R_{121}$ pouvant être identiques ou différents).

2. Procédé suivant la revendication 1, dans lequel le corticoïde (I) non saturé en position 16 est choisi dans le groupe comprenant des $\Delta^4$-3-céto-stéroïdes

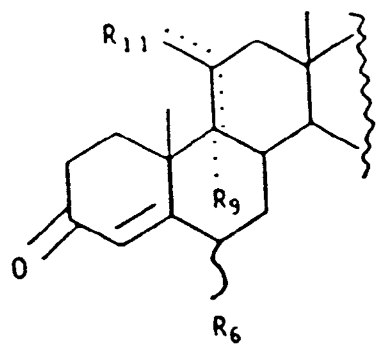

(A)

des $\Delta^{1,4}$-3-céto-stéroïdes

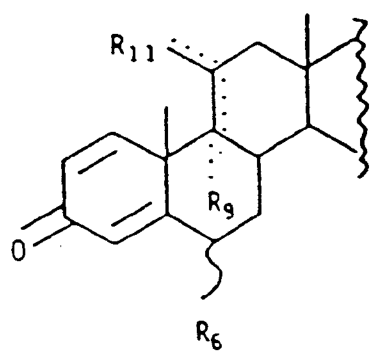

(B)

et des 3β-hydroxy-$\Delta^5$-stéroïdes

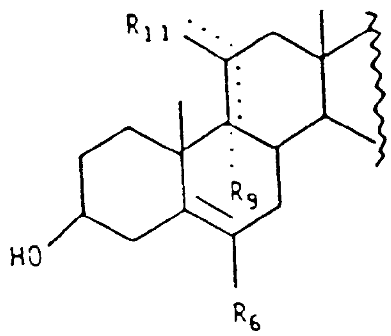

(C)

et les formes protégées en $C_3$ du 3β-hydroxy-$\Delta^5$-(C)-stéroïde

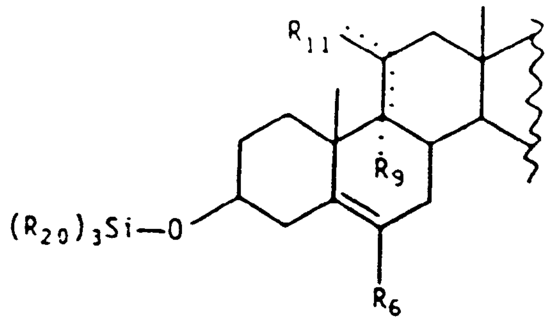

(Cb)

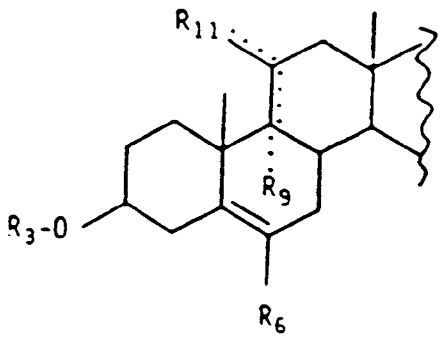

(Cc)

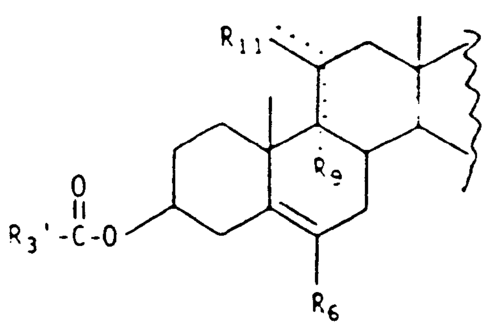

(Cd)

où

$R_3$ est un groupe alkyle ayant 1 à 3 atomes de carbone, un groupe TMS, THP ou EEE,

$R_3'$ est un groupe alkyle ayant 1 à 5 atomes de carbone ou phényle,

$R_6$ est un atome d'hydrogène ou de fluor ou un groupe méthyle,

$R_9$ ne représente rien ou représente un atome d'hydrogène, de fluor ou d'oxygène qui donne au noyau C la forme

a) $\Delta^{9(11)}$ lorsque $R_9$ ne représente rien et

b) 9β,11β-époxyde lorsque $R_9$ et $R_{11}$ pris ensemble représentent un atome d'oxygène,

$R_{11}$ est un atome d'hydrogène ou un atome d'oxygène, deux atomes d'hydrogène ou un groupe α- ou

β-hydroxyle ou son éther de triméthylsilyle qui donne au noyau C la forme

a) $\Delta^{9(11)}$ lorsque $R_{11}$ est un atome d'hydrogène,

b) 9β,11β-époxyde lorsque $R_9$ et $R_{11}$ pris ensemble représentent un atome d'oxygène et ... entre $C_{11}$ et $R_{11}$ représente une liaison simple, et

c) une cétone lorsque $R_{11}$ est un atome d'oxygène et ... entre $C_{11}$ et $R_{11}$ est une double liaison,

R20 est un groupe alkyle ayant 1 à 4 atomes de carbone ou phényle, les groupes $R_{20}$ pouvant être identiques ou différents,

R21 est un atome d'hydrogène, un groupe -CO-$R_{21}'$ ou -Si($R_{121}$)$_3$,

$R_{21}'$ est un groupe alkyle ayant 1 à 4 atomes de carbone ou phényle,

$R_{121}$ est un groupe alkyle ayant 1 à 4 atomes de carbone ou phényle, les groupes $R_{121}$ pouvant être identiques ou différents,

~ signifie que le groupe attaché peut être en configuration α ou β ;

... désigne une liaison simple ou double ;

TMS désigne le groupe triméthylsilyle ;

THP est le groupe tétrahydropyrannyle ;

EEE se réfère à l'éther de (1-éthoxy)éthyle ($-O-CH(CH_3)OCH_2CH_3$].

3. Procédé suivant la revendication 1, dans lequel l'agent de méthylation est choisi dans le groupe comprenant des composés de formule $CH_3Cu$, $(CH_3)_2CuM$ ou $CH_3MgQ$ où M est un ion lithium ou magnésium et Q est un atome de chlore, de brome ou d'iode.

4. Procédé suivant la revendication 3, dans lequel l'agent de méthylation est $CH_3MgQ$.

5. Procédé suivant la revendication 1, dans lequel le catalyseur au cuivre est un sel cuivrique choisi dans le groupe comprenant l'acétate cuivrique, le chlorure cuivrique, le sulfate cuivrique ou un sel cuivreux choisi dans le groupe comprenant le chlorure cuivreux, le bromure cuivreux, l'iodure cuivreux et le cyanure cuivreux et leurs complexes.

6. Procédé suivant la revendication 1, dans lequel le catalyseur au cuivre est l'acétate cuivrique.

7. Procédé suivant la revendication 1, dans lequel l'agent de silylation est choisi dans le groupe comprenant $(R_{20})_3$-Si-E, bistriméthylsilylacétamide, où E est un atome de chlore, de brome ou d'iode ou un groupe -NRαRβ dans lequel Rα et Rβ peuvent être identiques ou différents et sont des radicaux alkyle ayant 1 à 5 atomes de carbone ou phényle et Rα et Rβ peuvent être liés ou cyclisés en un noyau avec ou sans atome d'oxygène ou atome d'azote additionnel et $R_{20}$ est tel que défini dans la revendication 1.

8. Procédé suivant la revendication 1, dans lequel l'agent de silylation est le chlorure de triméthylsilyle.

9. Procédé suivant la revendication 1, dans lequel le corticoïde (I) non saturé en position 16 est choisi dans le groupe comprenant le 21-acétate de 21-hydroxyprégna-1,4,9(11),16-tétraène-3,20-dione ou le 21-acétate de 9β,11β-époxy-21-hydroxyprégna-1,4,16-triène-3,20-dione.

10. Procédé suivant la revendication 1, dans lequel l'agent de silylation est présent lorsque l'énolate intermédiaire est formé.